# FASCICULE DE BREVET EUROPEEN

(11) **EP 1 333 804 B1**
(45) Date de publication et mention de la délivrance du brevet: **21.03.2007**
(21) Numéro de dépôt: 01993454.6
(22) Date de dépôt: 13.11.2001
(51) Int. Cl.: A61K 8/66

(54) **COMPOSITIONS COMPRENANT UNE BETA-GALACTOSIDASE, UNE N-ACETYL-GLUCOSAMINIDASE ET UNE N-ACETYL-GALACTOSAMINIDASE, LESDITES COMPOSITIONS NE COMPRENANT PAS DE PROTEASE**
ZUSAMMENSETZUNGEN ENTHALTEND BETA-GALACTOSIDASE, N-ACETYL-GLUCOSAMINIDASE UND N-ACETYL-GALACTOSAMINIDASE, DIE KEINE PROTEASE ENTHALTEN
COMPOSITIONS COMPRISING BETA-GALACTOSIDASE, N-ACETYL-GLUCOSAMINIDASE AND N-ACETYL-GALACTOSAMINIDASE, SAID COMPOSITIONS NOT COMPRISING PROTEASE

(30) Priorité: 13.11.2000 FR 0014556
(43) Date de publication de la demande: 13.08.2003
(73) Titulaire: L'OREAL S.A., 75008 Paris Cedex (FR)
(72) Inventeur: MEHUL, Bruno, F-92800 Villejuif (FR); BERNARD, Dominique, F-75015 Paris (FR)
(74) Mandataire: Desaix, Anne
(86) Numéro de dépôt international: PCT/FR2001/003551
(87) Numéro de publication internationale: WO 2002/038122

(56) Documents cités:
- WO-A-97/47202
- DE-A- 19 649 096
- FR-A- 2 732 593
- US-A- 5 395 541

## Description

La présente invention a trait à des compositions, favorisant la desquamation. Elle porte en particulier sur des compositions exemptes de protéases et comprenant au moins une association de β-galactosidase, N-acétyl-glucosaminidase et N-acétyl-galactosaminidase, et éventuellement un activateur des glycosidases et leurs utilisations pour lutter contre le vieillissement cutané ou contre les peaux sèches.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes, cellules mortes qui constituent la dernière couche de l'épiderme, c'est à dire la couche la plus externe encore appelée stratum corneum qui s'éliminent par desquamation. Cette perte en surface est compensée par la migration de cellules de l'assise basale vers la surface de l'épiderme. Il s'agit du renouvellement perpétuel de la peau.

Une peau jeune renouvelle ses couches superficielles toutes les deux à trois semaines, alors qu'une peau mature peut prendre deux fois plus de temps. Une des conséquences de ce ralentissement du renouvellement épidermique est l'impression de peau sèche. En effet, plus le processus de renouvellement est long, plus l'hydratation naturelle à la surface de la peau diminue.

De plus, plus la desquamation est ralentie, plus la peau prend un aspect mat et perd de son éclat et de sa fraîcheur.

Une élimination forcée de la couche cornée accélère le renouvellement épidermique, permet de lutter contre le vieillissement cutané et la peau retrouve alors un aspect jeune et frais ainsi qu'une meilleure hydratation. La peau perd ainsi son aspect mat et le teint s'en trouve amélioré.

Le vieillissement cutané, résultant d'effets sur la peau de facteurs intrinsèques ou extrinsèques, se traduit par l'apparition de rides et ridules, par le jaunissement de la peau, qui développe notamment un aspect parcheminé, accompagné éventuellement de l'apparition de taches pigmentaires, par la désorganisation des fibres d'élastine et de collagène entraînant une perte d'élasticité, de souplesse et de fermeté et par l'apparition de télangiectasies.

Certains de ces signes du vieillissement sont plus particulièrement liés au vieillissement intrinsèque ou physiologique, c'est-à-dire au vieillissement « normal » lié à l'âge ou chronobiologique, alors que d'autres sont plus spécifiques du vieillissement extrinsèque, c'est-à-dire du vieillissement provoqué d'une manière générale par l'environnement ; il s'agit plus particulièrement du photovieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et/ou tannée, et des changements histopathologiques tels qu'une excessive accumulation de matière élastique dans le derme supérieur et une dégénérescence des fibres de collagène.

Les dégradations protéolytiques observées lors du processus de desquamation seraient induites par des protéases synthétisées par le stratum corneum, en particulier l'enzyme apparentée à la chymotrypsine du stratum corneum (SCCE). Il a été par ailleurs montré que des glycosidases d'origine lysosomiale sont externalisées au cours de la différenciation terminale de l'épiderme (Nemanic *et al.,* J. of Invest. Dermat., 1983, 81 :28-33). Néanmoins, la caractérisation des substrats de ces glycosidases au niveau du stratum corneum n'a que peu progressé et le rôle biologique de ces glycosidases n'a pas été clairement élucidé.

Il a été observé que la présence de résidus saccharidiques sur les protéines protège les structures desmosomales et les glycoprotéines des coméosomes d'une dégradation protéolytique induite artificiellement par l'addition de protéases (Walsh et Chapman (1990), Arch Dermatol Res 282:304-310).

De cette observation, il résulte l'hypothèse que les glycosidases endogènes pourraient favoriser l'action des protéases au niveau du stratum corneum et ainsi accélérer le phénomène de desquamation.

Il est aussi possible que les sucres générés par l'activité glycosidase endogène entraînent directement une réponse cellulaire conduisant au relarguage des cornéocytes.

La mise au point de cosmétiques favorisant le phénomène de desquamation par leur application sur la peau permet donc de lutter contre les peaux sèches et le vieillissement cutané en particulier. De nombreux exemples de compositions cosmétiques favorisant la desquamation sont décrits dans l'état de la technique. Citons en particulier des compositions contenant de l'acide rétinoïque telles que décrites dans le brevet US-A-4,603,146, des compositions contenant des α- ou β-hybroxydes (voir par exemple les demandes EP-A-413 528 ou WO-A-93/10756). Ces composés présentent néanmoins des effets secondaires consistant en des picotements, des tiraillements, des échauffements et des rougeurs désagréables pour l'utilisateur. Des exemples de compositions comprenant essentiellement des protéases ou une combinaison de protéases et glycosidases sont décrits dans l'état de la technique (FR-A-2 732 593, Bieurope SA ; PCT WO 93/19732, Unilever PLC ; PCT WO 95/07687, Unilever PLC). Cependant, l'utilisation de protéases dans des compositions topiques comporte des risques toxicologiques et peut notamment entraîner des réactions allergiques et des phénomènes d'irritation.

L'invention résulte d'études de l'effet de glycosidases exogènes sur le phénomène de desquamation. Ces études ont été réalisées à l'aide de la mise au point d'un test *in vitro* de desquamation adapté au criblage de molécules. Ces études ont ainsi permis de montrer que, de manière surprenante, une augmentation de l'activité glycosidase au niveau du stratum corneum par l'addition de glycosidases exogènes spécifiques, le cas échéant en association avec un produit capable de stimuler l'activité glycosidase endogène est suffisante en l'absence d'addition de protéases exogènes pour favoriser le phénomène de desquamation.

Par conséquent, l'invention porte sur des compositions comprenant, à titre de principe actif, au moins une association de β-galactosidase, N-acétyl-glacosaminidase, et N-acétyl-galactosaminidase favorisant la desquamation, ladite composition ne comprenant pas de protéase.

Les autres glycosidases comprises dans les compositions selon l'invention sont toutes les enzymes susceptibles d'avoir comme substrat les protéoglycanes, les glycolipides et en général les glycoconjugués du stratum corneum. De telles enzymes sont les sialidases comme par exemple les neuraminidases, les mannosidases, les galactosidases, les glucosidases, les chondroïtinases, les glucuronidases ou encore les hyaluronidases.

Elles peuvent aussi être toutes glycosidases favorisant la desquamation, sélectionnées par tout test d'efficacité des glycosidases dans la desquamation (Lundström A. et Egelrud T., Arch. Dermatol. Res. (1990), 282 : 234-237), ou encore par le test comprenant les étapes suivantes :
a) la récupération d'échantillons du stratum corneum à partir d'un individu ;
b) la fixation de l'échantillon sur un support ;
c) la mise en contact de l'échantillon fixé sur un support avec une solution tamponnée contenant la glycosidase à tester ;
d) la récupération du matériel insoluble libéré dans la solution tamponnée ;
e) le dosage des kératines présentes dans le matériel insoluble et leur quantification relative par rapport à un test contrôle sans glycosidase.

Par le présent test, les kératines dosées à l'étape e) dans le matériel insoluble reflètent la quantité de cornéocytes libérés. Il permet ainsi de tester l'effet « prodesquamant » d'un produit et plus particulièrement des glycosidases.

Ces autres glycosidases comprennent aussi les cellulases pour lesquelles aucun substrat endogène au niveau du stratum corneum n'est connu.

Les glycosidases pour lesquelles un effet prodesquamant a été mis en évidence à l'aide des études i*n vitro* sont *:* La N-glycanase, les cellulases, la β-glucosidase. Dans l'invention, une association de trois glycosidases, la β-galactosidase, la N-acétyl-glucosaminidase et la N-acétyl-galactosaminidase, est comprise dans la composition.

Les glycosidases utilisées dans les compositions selon l'invention peuvent être purifiées à partir d'extraits de protéines synthétisées par les cellules du stratum corneum ou peuvent être des glycosidases naturellement synthétisées par des micro-organismes. Elles peuvent aussi être des glycosidases recombinantes produites en système hétérologue.

De manière générale, une composition selon l'invention comprend de 0,001 à 15% en poids de glycosidases, de manière préférée de 0,01 à 10% et mieux de 0,05 à 5% en poids par rapport au poids total de la composition.

L'invention résulte de l'observation que l'augmentation de l'activité glycosidase au niveau du stratum corneum est suffisante pour favoriser la desquamation. Par conséquent, l'invention porte aussi sur des compositions cosmétiques comprenant un produit capable de stimuler l'activité des glycosidases. Un produit capable de stimuler l'activité des glycosidases est un produit qui permet d'augmenter la vitesse de la réaction enzymatique, mesurée par exemple par l'augmentation de la quantité de substrats digérés par unité de temps lorsque ledit produit est ajouté dans le milieu réactionnel. Un exemple de produit capable de stimuler l'activité β-glucosidase est le 1-O-méthyl-β-D-glucopyranoside. Ces activateurs représentent entre 0,01% et 10% du poids total de la composition, de manière préférée entre 0,1 et 1% du poids total de la composition.

Dans les compositions utilisables selon l'invention et définies précédemment, les glycosidases peuvent être associées à d'autres actifs ayant des propriétés desquamantes connues, commes les hydroxy-acides, les α- ou β-céto-acides, les rétinoïdes, certains acides sulfoniques ou certains carbohydrates tels que ceux définis dans la demande de brevet (L'Oreal, « utilisation de carbohydrates pour favoriser la desquamation de la peau » WO-A-97 12597). Une telle association permet de diminuer la concentration active de ces actifs du fait des effets additifs. On peut ainsi obtenir une composition moins irritante et moins toxique, ainsi qu'une composition plus efficace que celles de l'art antérieur n'utilisant que ces actifs.

Les hydroxyacides peuvent être par exemple des α-hydroxyacides ou des β-hydroxyacides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Ces hydroxyacides sont notamment les acides glycolique, lactique, malique, tartrique, citrique et de manière générale les acides de fruits, les acides hydroxy-2 alcanoïque, mandélique, salicylique, ainsi que leurs dérivés alkylés ou acylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol ainsi que leurs sels, ou encore le rétinal.

A titre d'exemple, les hydroxyacides, les céto-acides et les rétinoïdes peuvent être introduits dans les compositions utilisées selon l'invention en une quantité représentant de 0,01 à 5% en poids total de la composition et mieux de 0,1 à 3%.

La composition utilisée selon l'invention contient un milieu cosmétiquement ou dermatologiquement acceptable, c'est-à-dire un milieu compatible avec la peau, les ongles, les muqueuses, les tissus et les cheveux. Selon un mode préféré de réalisation de l'invention, la composition a un pH permettant une activité optimale des glycosidases utilisées et de préférence proche de celui de la peau, compris entre 4 et 7. La composition comprenant les glycosidases est de préférence appliquée par voie topique sur le visage, le cou, les cheveux, les muqueuses et les ongles ou tout autre zone cutanée du corps.

Une composition cosmétique selon l'invention se présente de préférence sous une forme appropriée pour une administration par voie topique et pour contenir des actifs de types enzymes qui présentent une instabilité en milieu aqueux et pour une application topique. Elle se présente notamment sous forme de solutions hydroalcooliques ou huileuses, de dispersions du type lotion ou sérum, de gels anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de micro-émulsions, ou encore de micro-capsules, de micro-particules, ou de dispersions vésiculaires de type ionique et/ou non ionique. Ces compositions sont préparées selon les méthodes usuelles. Une forme préférée et spécialement adaptée pour des compositions comprenant des actifs enzymes est une forme de type E/H/E telle que décrite dans la demande EP 0 779 071 (L'Oréal).

Les compositions selon l'invention peuvent être également utilisées pour les cheveux sous forme de solutions alcooliques ou hydroalcooliques, ou sous forme de crèmes, de gels, d'émulsions, de mousses.

Les quantités des différents constituants des compositions utilisées selon l'invention sont celles classiquement utilisées dans les domaines considérés.

Ces compositions constituent notamment des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions peuvent également consister en des préparations solides constituant des savons ou des pains de nettoyage.

De façon connue, la composition utilisée selon l'invention peut contenir également des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges et les matières colorantes. Les quantités de ces différents adjuvants sont celles classiquement utilisées dans les domaines considérés, et par exemple de 0,01% à 20% du poids total de la composition. Bien entendu, l'homme de l'art veillera à choisir ce ou ces éventuels additifs et/ou leurs quantités de manière telle que les propriétés avantageuses attachées intrinsèquement à la composition conforme à l'invention, en particulier les activités enzymatiques des glycosidases, ne soient pas, ou substantiellement pas, altérées par la ou les adjonctions envisagées.

Comme huiles utilisables dans l'invention, on peut citer les huiles minérales (huile de vaseline), les huiles végétales (huile de karité, huile d'amande douce), les huiles animales, les huiles de synthèse, les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émultionnants utilisables dans l'invention, on peut citer le Polysorbate 60 et le stéarage de sorbitane vendus respectivement sous les dénominations commerciales Tween 60 et Span 60 par la Société ICI. On peut y ajouter des co-émulsionnants tels que le PPG-3 myristyl éther vendu sous la dénomination commerciale Emcol 249-3K par la société Witco.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles (xanthane) et les argiles, et, comme gélifiants lipophiles, on peut citer les argiles modifiées comme les bentones, les sols métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, les acides gras essentiels, les céramides, les huiles essentielles.

En vue de lutter efficacement contre le photovieillissement, il est en outre possible d'ajouter à la composition utilisée selon l'invention un ou plusieurs filtres solaires complémentaires, actifs dans l'UVA et/ou UVB, hydrophiles ou lyophiles, comportant éventuellement une fonction sulfonique.

Le filtre solaire est de préférence choisi parmi les filtres organiques et/ou les filtres minéraux.

Comme filtres organiques, on peut notamment citer les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665 ou encore les filtres organiques décrits dans la demande de brevet EP-A 0 487 404.

Comme filtres minéraux, on peut notamment citer des pigments ou bien encore des nanopigments (taille moyenne des particules primaires : généralement entre 5nm et 10 nm, de préférence entre 10 et 50 nm) d'oxydes métalliques enrobés ou non, comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particlier décrits dans les demandes de brevets EP-A-0 518 772 et EP-A-0 518 773.

Comme exemples de filtres solaires complémentaires actifs dans l'UV-A et/ou l'UV-B, on peut citer :
- l'acide p-aminobenzoïque,
- le p-aminobenzoate oxyéthyléné (25 mol),
- le p-diméthylaminobenzoate de 2-éthylhexyle,
- le p-aminobenzoate d'éthyle N-oxypropyléné,
- le p-aminobenzoate de glycérol,
- le salicylate d'homomenthyle,
- le salicylate de 2-éthylhexyle,
- le salicylate de triéthanolamine,
- le salicylate de 4-isopropylbenzyle,
- le 4-ter-butyl-4'-méthoxy-dibenzoylméthane (PARSOL 1789 de GIVAUDAN ROURE)
- le p-méthoxycinnamate de 2-éthylhexyle (PARSOL MCX de GIVAUDAN ROURE)
- le 4-isopropyl-dibenzoylméthane (EUSOLEX 8020 de MERCK),
- l'anthranilate de menthyle,
- le 2-éthylhexyl-2-cyano-3,3'-diphénylacrylate, (UVINUL N539 de BASF),
- l'éthyl-2-cyano-3,3'-diphénylacrylate,
- l'acide 2-phényl benzimidazole 5-sulfonique et ses sels,
- le 3-(4'-triméthylammonium)-benzylidèn-bornan-2-on-méthylsulfate,
- le 2-hydroxy-4-méthoxybenzophénone (UVINUL MS 40 de BASF),
- le 2-hydroxy-4-méthoxybenzophénone-5-sulfonate (UVINUL MS 40 de BASF),
- le 2,4-dihydroxybenzophénone (UVINUL 400 de BASF),
- le 2,2',4,4'-tétrahydroxybenzophénone (UVINUL D 50 de BASF),
- le 2,2'-dihydroxy-4,4'-diméthoxybenzophénone (HELISORB II de NORQUAY),
- le 2-hydroxy-4-n-octoxybenzophénone,
- le 2-hydroxy-4-méthoxy-4'-méthylbenzophénone,
- l'acide α-(2-oxoborn-3-ylidène)-tolyl-4-sulfonique et ses sels,
- le 3-(4'-sulfo)benzylidèn-bornan-2-one et ses sels,
- le 3-(4'-méthylbenzylidène)-d,l-camphre,
- le 3-benzylidène-d,l-camphre,
- l'acide benzène 1,4-di(3-méthylidène-10-camphosulfonique) et ses sels (MEXORYL SX de CHIMEX),
- l'acide urocanique,
- le 2,4-6-tris-[p-(2'-éthylhexyl-1'-oxycarbonyl)anilino]-1,3,5-triazine,
- le 2-[p-(tertiobutylamido)anilino]-4,6-bis [p-(2'-éthylhexyl-1'-oxycarbonyl)anilino]-1,3,5-triazine,
- le 2,4-bis{[4-2-éthyl-hexyloxyl]-2-hydroxyl-phenyl}-6-(4-méthoxy-phenyl)-1,3,5-triazine,
- le polymère de N-(2 et 4)-[2-oxoborn-3-ylidèn)méthyl)benzyl]-acrylamide,
- l'acide 4,4-bis-benzimidazolyl-phénylèn-3,3',5,5'-tétrasulfonique et ses sels,
- le 2,2'-méthylèn-bis-[6-(2H-benzotriazol-2-yl)-4-(1,1,3,3-tétraméthylbutyl) phénol],
- les polyorganosiloxanes à fonction malonate.

L'invention porte aussi sur un procédé de traitement cosmétique, par l'application des compositions telles que définies ci-dessus, selon la technique d'utilisation habituelle de ces compositions. Par exemple : application de crèmes, de gels, de sérums, de pommades, de lotions, de laits sur la peau, le cuir chevelu, les ongles et/ou les muqueuses.

Les compositions favorisant la desquamation sont adaptées pour le traitement des peaux sèches ou pour lutter contre le vieillissement cutané.

Par conséquent, l'invention porte aussi sur l'utilisation de compositions telles que décrites précédemment pour le traitement des peaux sèches ou le traitement et/ou la prévention du vieillissement cutané.

La partie qui suit présente les résultats de l'étude *in vitro* de l'effet de différentes glycosidases sur la desquamation. Elle présente par ailleurs des exemples de formulation de compositions selon l'invention sans en restreindre la portée des revendications.

### PARTIE EXPERIMENTALE

### Légendes des figures

La figure 1 est un histogramme illustrant l'effet de l'urée à 5 % sur le détachement des cornéocytes. Les valeurs sont représentées en pourcentage du niveau de détection des kératines par rapport au contrôle négatif contenant le tampon de base PBS (100 %).
La figure 2 illustre l'effet dose-réponse de la N-glycanase sur le détachement des cornéocytes en comparaison avec l'urée (par immunoblot).
La figure 3 est un graphe illustrant l'effet de la N-glycanase à trois concentrations différentes sur le détachement des cornéocytes. Les valeurs sont des moyennes de 5 expériences indépendantes exprimées en pourcentage du niveau de détection des kératines par rapport au contrôle négatif contenant le tampon de base (100%).
La figure 4 illustre l'effet sur la desquamation de certaines glycosidases et de certaines cellulases commercialisées par Clonezyme par immunoblot.
La figure 5 est un graphe illustrant l'effet de la cellulase sur le détachement des cornéocytes en comparaison avec le tampon de base. Les valeurs sont représentées en pourcentage du niveau de détection des kératines par rapport au contrôle négatif contenant le tampon de base (100%).
La figure 6 est un graphe illustrant l'effet d'un mélange de glycosidases sur le détachement des cornéocytes. Les valeurs sont représentées en pourcentage du niveau de détection des kératines par rapport au contrôle négatif contenant le tampon de base (100%).

### 1. Test de «desquamations» in vitro adapté au criblage de molécules et validation par l'étude de différentes glycosidases

### 1.1 Mise au point d'un test de desquamation in vitro adapté au criblage de molécules

### 1.1.1 Méthode

Pour la récupération d'échantillons des couches superficielles du stratum corneum, le test utilise la méthode du blenderm-vernis réalisée sur les mollets. Du vernis est appliqué directement sur la peau puis, après 10 minutes, les couches superficielles de stratum corneum sont prélevées avec de l'adhésif de type blenderm (3M). Le filtre contenant l'échantillon est ensuite ajusté sur une plaque de microtitration de 96 puits en Téflon et fixé sur une base permettant l'étanchéité entre les puits.

90 µl de tampon de base (PBS, pH 6,5, 0,1 % acide de sodium, 0,05 % tween 20) contenant les enzymes ou produits testés sont ajoutés par puits. Chaque détermination est réalisée sur 3 à 4 puits répartis différemment sur la plaque après un temps d'incubation compris entre 1 heure et 48 heures à température comprise entre 20 et 37°C. 40 µl de chaque puits traité dans les mêmes conditions sont mélangés et filtrés sur tamis de porosité de 0,22 µm, afin d'éliminer les protéines solubles et retenir ainsi les cornéocytes libérés. Les cornéocytes sont repris dans 30 µl de tampon de base en présence de DTT et chauffés pendant 15 minutes à 80°C. Les échantillons sont ensuite centrifugés pour éliminer le matériel insoluble, et analysés par SDS-PAGE et/ou Western Blot à l'aide d'anticorps monoclonaux anti-kératine K10 (kératines suprabasales). Les kératines sont détectées après coloration des protéines au nitrate d'argent (à un poids moléculaire apparent d'environ 55-65 kDa). L'utilisation d'un anticorps monoclonal anti-K10 permet de confirmer la présence de kératines suprabasales solubilisées.

### 1.1.2 Résultats

L'analyse des fractions passées par le tamis de porosité 0,22 µm (contenant les protéines solubles) ne contient pas de protéines majoritairement représentées à 55-65 kDa correspondant à la zone de migration des kératines. En revanche, l'analyse de la fraction retenue par le filtre permet l'identification des kératines. Ceci confirme que l'analyse des kératines par immunoblot reflète bien la présence de cornéocytes, les kératines des cornéocytes étant liées au matériel insoluble. La quantification des kératines détectées permet donc d'obtenir une estimation de la quantité de cornéocytes libérés au cours de l'incubation et donc une évaluation de l'effet prodesquamant d'un produit.

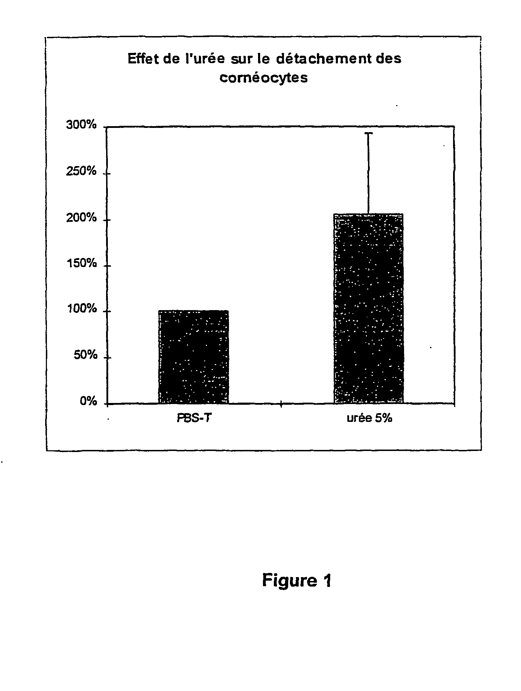

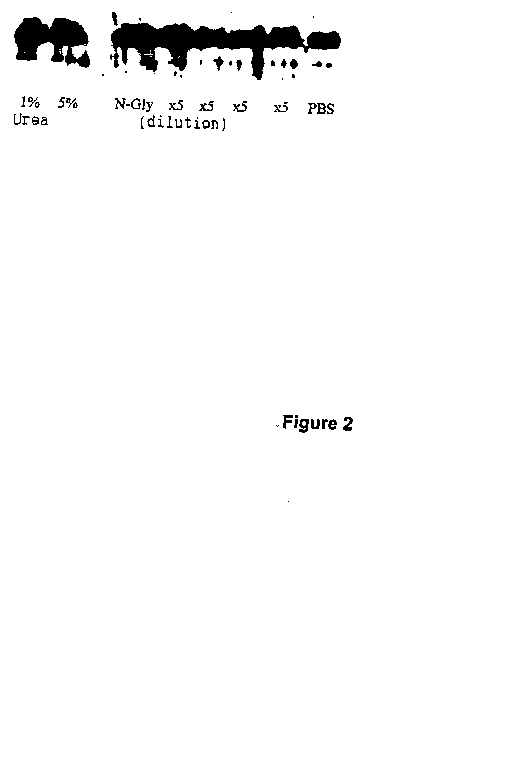

L'urée est un composant connu pour son action positive sur la desquamation. Afin de valider le test, cette molécule a été utilisée comme contrôle positif à des concentrations de 1 % et 5% (en comparaison avec le témoin négatif constitué de tampon de base uniquement). Les résultats sont présentés dans les figures 1 et 2. Les données moyennes des tests réalisés sur 4 expériences indépendantes montrent clairement un effet de l'urée à 5% (200% +/- 100% de kératine mesurée par rapport au 100% mesuré sans urée).

Ces résultats permettent de valider l'utilisation de ce test pour le criblage de molécules susceptibles de favoriser la desquamation.

### 1.2 Etudes de l'effet de différentes glycosidases sur la desquamation

### 1.2.1 Etude de la N-glycanase (Endo-F, de Flavobacterium meningosepticum) sur la desquamation

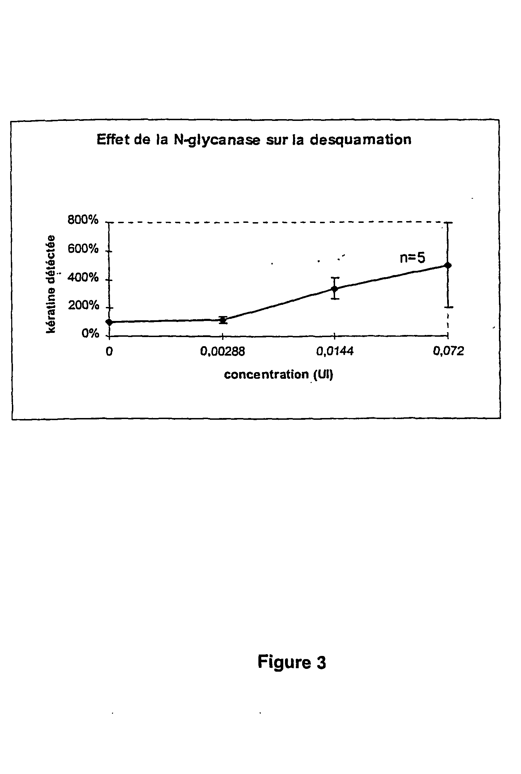

La N-glycanase (endo. F) permet d'éliminer toutes les chaînes N-liées portées par les glycoprotéines. La N-glycanase utilisée est celle commercialisée par Boehringer. Les résultats présentés dans les figure 2 et 3 montrent clairement un effet de la N-glycanase sur le détachement des cornéocytes.

### 1.2.2 Etude de glycosidases recombinantes thermostables sur la desquamation

Différentes glycosidases commercialisées par CloneZyme^{™} ont été testées pour leur capacité à favoriser la desquamation. Le tableau 1 ci-dessous présente les spécificités de substrats des différentes glycosidases testées.

**Tableau 1**

| | GLY-01 | GLY-02 | GLY-03 | GLY-04 | GLY-05 | GLY-06 | GLY-07 | GLY-08 | GLY-09 | GLY-10 |
|---|---|---|---|---|---|---|---|---|---|---|
| β-D-cellobiose | - | ++ | ε | ε | | + | + | ε | ε | - |
| β-D-galactose | - | ++ | ε | + | + | ε | ε | + | - | - |
| α-D-glucose | - | - | - | - | - | - | - | - | - | - |
| β-D-glucose | ++ | ++ | ++ | ++ | ++ | ++ | ++ | ++ | ++ | - |
| β-N-acetyl-D-glucosaminide | - | - | - | - | - | - | - | - | - | - |
| β-D-fucose | - | ++ | ++ | ++ | ++ | ++ | ++ | + | ++ | - |
| β-L-fucose | - | - | - | - | - | - | - | - | - | - |
| β-D-glucuronide | - | - | - | - | - | - | ε | - | - | - |
| α-D-gatactose | - | - | - | - | - | - | - | - | - | + |
| β-D-mannose | - | ε | - | - | - | + | + | - | + | - |
| α-D-mannose | - | - | - | - | - | - | - | - | - | - |
| β-D-xylose | ε | + | - | ε | ε | ε | ε | ε | ε | - |
| α-L-arabinofuranoside | - | - | - | - | - | - | - | - | - | - |
| α-L-arabinopyranoside | - | + | ε | ε | ε | ε | ε | ε | ε | - |
| β-D-lactose | - | + | ε | - | - | + | ε | - | - | - |
| α-L-rhamnose | - | - | - | - | - | - | - | - | - | - |
| α-D-N-acetylneuramide | - | - | - | - | - | - | - | - | - | - |
| β-D-N-acetylchitobioside | - | - | - | - | - | - | - | - | - | - |
| α-L-fucose | - | - | - | - | - | - | - | - | - | - |

| | | | | | | | | | | |
|---|---|---|---|---|---|---|---|---|---|---|
| (- : pas d'activité ; ε, +,++ : activité spécifique) | | | | | | | | | | |

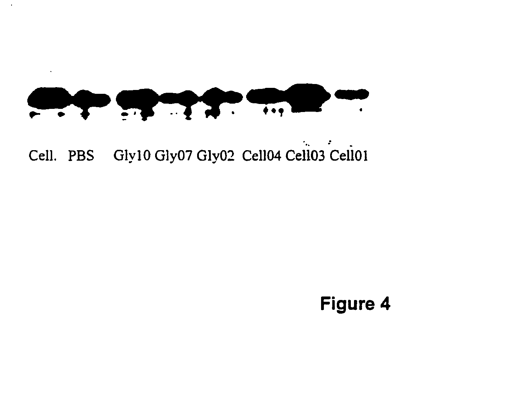

Un des intérêts de ces glycosidases est leur disponibilité relativement aisée et leur stabilité vis-à-vis des variations de température. Elles sont par conséquent plus particulièrement aptes à entrer dans la préparation de compositions appropriées pour l'administration topique. Par l'identification des kératines après coloration au nitrate d'argent des gels SDS-PAGE et immunodétection, les résultats présentés dans la figure 4 montrent que ces glycosidases favorisent la desquamation. La spécificité de ces glycosidases vis-à-vis de leurs substrats est plus ou moins étroite. Les résultats montrent un optimum d'efficacité avec la glycosidase Gly 10, avec par ordre d'efficacité, Gly10 > Gly07 > Gly02.

### 1.2.3 Etude de l'effet des cellulases sur la desquamation

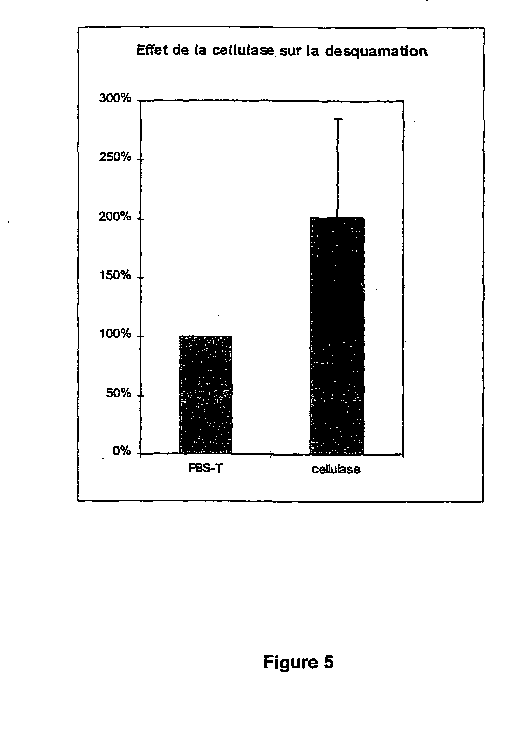

Les cellulases coupent les liaisons β1-4 situées entre deux glucoses internes (endoglycosidases). La présence de cellulose ou de structures proches n'a pas été décrite dans l'épiderme humain. Néanmoins, il reste possible que des structures saccharidiques au niveau du stratum corneum constituent des cibles de cellulases. L'effet de la cellulase commercialisée par Boehringer a été testée pour sa capacité à favoriser la desquamation. Les résultats présentés dans la figure 5 montrent de manière surprenante un effet important (similaire à l'urée) de la cellulase sur la desquamation. Des résultats similaires ont été obtenus avec plusieurs cellulases recombinantes commercialisées par CloneZyme^{™}, avec par ordre d'efficacité cell03 > cell04, cell01 (figure 4).

Le tableau 2 présente la spécificité de substrats des différentes cellulases testées vis-à-vis des glycosides.

**Tableau 2**

| | **CELLULASES (CLONEZYME)** | | | | | | |
|---|---|---|---|---|---|---|---|
| **Substrat** | CEL-001 | CEL-002 | CEL-003 | CEL-004 | CEL-005 | CEL-006 | CEL-007 |
| β-D-cellobiose | ++ | ++ | - | ++ | - | - | - |
| β-D-galactose | + | - | - | - | - | - | - |
| α-D-glucose | - | - | - | - | - | - | - |
| β-D-glucose | ++ | - | - | - | - | - | - |
| β-N-acetyl-D-gfucosaminide | - | - | - | - | - | - | - |
| β-D-fucose | ++ | - | - | - | - | - | - |
| β-L-fucose | - | - | - | - | - | - | - |
| β-D-glucuronide | - | - | - | - | - | - | - |
| α-D-galactose | - | - | - | + | - | ++ | - |
| β-D-mannose | ++ | - | ++ | ++ | - | - | - |
| α-D-mannose | - | - | - | - | - | - | - |
| β-D-xylose | + | - | - | - | - | - | - |
| α-L-arabinofuranoside | - | - | - | - | - | - | - |
| α-L- arabinopyranoside | + | - | - | - | - | - | - |
| β-D-lactose | ++ | ++ | - | ++ | - | - | - |
| α-L-rhamnose | - | - | - | - | - | - | - |
| α-D-N-acetylneuramide | - | - | - | - | - | - | - |
| β-D-N-acetylchitobioside | - | - | - | - | - | - | - |
| α-L-fucose | - | - | - | - | - | - | - |

| | | | | | | | |
|---|---|---|---|---|---|---|---|
| (+ : 1-10% activité relative ; 10-100% activité relative) | | | | | | | |

### 1.2.4 Effets de différentes exo-glycosidases et des O-glycanases

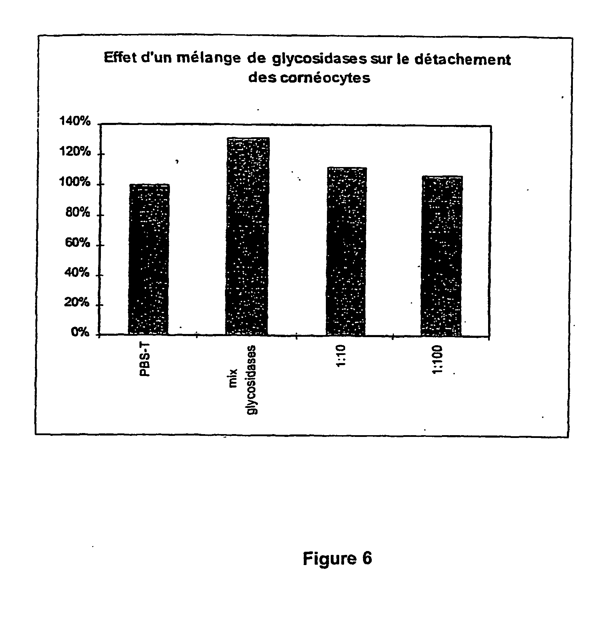

Certaines exo-glycosidases ont été testées pour leur effet sur la desquamation. En particulier, 40 U de β-galactosidase, d'α-fucosidase, 1 U d'N-acétyl-glucosaminidase, 0,5 U d'α-galactosidase, 0,3 U d'α-mannosidase, 0,2 U de β-mannosidase, 50 U de glucuronidase, 5 U de β-glucosidase et 5 U d'α-glucosidase ont été testées *in vitro.* Prises séparément, les exoglycosidases n'affectent que très faiblement la desquamation. La combinaison qui montre un effet significatif sur la desquamation est le mélange de β-galactosidase, N-acétyl-glucosaminidase et N-acétyl-galactosaminidase. La figure 6 montre l'effet de cette combinaison d'enzymes sur le détachement des cornéocytes.

La O-glycanase permet de cliver spécifiquement les chaines O-liée. La O-glycanase a été testée pour sa capacité à favoriser la desquamation. Les résultats obtenus en tests *in vitro* montrent que la O-glycanase ne semble pas ou peu affecter la desquamation (10% d'augmentation observée). De plus, l'utilisation simultanée de O-glycanase et de sialidase à large spectre d'action (*Arthrobacter ureafaciens*) ou de O-glycanase + N-glycanase et sialidase ne montre pas d'effet supérieur à celui observé avec la N-glycanase seule.

### 2. Exemples de formulations de compositions

### Composition 1 : Lait pour le visage (n'illustre pas l'invention)

- Huile de vaseline 7,0 g
- N-glycanase (Endo-F de Flavobacterium meningosepticum) 0,1 g
- Monostéarate de glycéryle, stéarate de polyéthylène glycol(100 OE) 3,0 g
- Polymère carboxyvinylique 0,4 g
- Alcool stéarylique 0,7 g
- Protéines de soja 3,0 g
- NaOH 0,4 g
- Conservateur qs
- Eau qsp 100 g

Cette composition se présente sous forme d'un lait pour le visage, ayant de bonnes propriétés cosmétiques, et étant doux et confortable à utiliser.

Le pH de la composition est d'environ 5,5.

### Composition 2 : Lotion (n'illustre pas l'invention)

- β-glucosidase 0,5g
- Palmitate d'éthyl-2 hexyle 10,0 g
- Cyclopentadiméthylsiloxane 20,0 g
- Butylène glycol 5,0 g
- Conservateur qs
- Eau qsp 100 g

Cette lotion, qui ne contient pas de tensioactif, favorise la desquamation de la peau.

### Composition 3 : Lait (n'illustre pas l'invention)

- Palmitate d'octyle 35,0 g
- Glygérine 2,0g
- N-acétyl-glucosaminidase 0,8 g
- Polymère réticulé acrylates C10-C30/alkylacrylates 0,1 g
- Triéthanolamine 0,1 g
- Acides aminés de blé 1,0 g
- Conservateur qs
- Eau qsp 100 g

Le lait obtenu, qui ne contient pas de tensioactif, possède de bonnes propriétés cosmétiques.

### Composition 4 : Gel pour le visage (n'illustre pas l'invention)

- Glycérine 10,0g
- N-acétyl-galactosidase 1,0 g
- Cocoamphodiacétate de disodium 10,0g
- Conservateur qs
- Eauqsp 100 g

Le gel obtenu possède de bonnes propriétés cosmétiques.

### Composition 5 : Gel nettoyant à l'eau (n'illustre pas l'invention)

- Butylène glycol 7,0 g
- Sarcosinate de lauroyl sodium 4,0 g
- β-glucosidase 1,0 g
- Triéthanolamine 0,8 g
- Carbomer 0,5 g
- Conservateur qs
- Eauqsp 100 g

Le gel obtenu possède de bonnes propriétés cosmétiques.

## Revendications

1. Utilisation pour favoriser la desquamation, d'une composition cosmétique comprenant, à titre de principe actif, au moins une association de β-galactosidase, N-acétyl-glucosaminidase et N-acétyl-galactosaminidase favorisant la desquamation, ladite composition ne comprenant pas de protéase.

2. Utilisation selon la revendication 1, **caractérisée en ce que** la composition comporte en outre une glycosidase choisie parmi les enzymes suivantes : la N-glycanase, les cellulases et/ou la β-glucosidase.

3. Utilisation selon la revendication 1 ou 2, **caractérisée en ce que** la composition comprend en plus un produit capable de stimuler l'activité glycosidase.

4. Utilisation selon l'une quelconque des revendications 1 à 3, **caractérisée en que** les glycosidases favorisant la desquamation représentent entre 0,001 à 15% du poids total de la composition, de manière préférée de 0,01 à 10% et mieux de 0,05% à 5%.

5. Utilisation selon la revendication 4, **caractérisée en qu'**un produit capable de stimuler l'activité glycosidase représente de 0,01 à 10% du poids total de la composition, de manière préférée de 0,1 à 1%.

6. Utilisation selon l'une quelconque des revendications 1 à 5, **caractérisée en ce qu'**au moins une glycosidase favorisant la desquamation est une glycosidase recombinante.

7. Utilisation selon l'une quelconque des revendications 1 à 6, **caractérisée en ce qu'**au moins une glycosidase est une glycosidase isolée du *stratum corneum.*

8. Utilisation selon l'une quelconque des revendications 1 à 7, **caractérisée en ce que** la composition comprend en plus au moins un autre actif ayant des propriétés favorisant la desquamation.

9. Utilisation selon l'une quelconque des revendications 1 à 8, **caractérisée en ce que** la composition est appropriée pour l'administration topique.

10. Utilisation d'une composition selon l'une quelconque des revendications 1 à 9 pour le traitement des peaux sèches ou le traitement et/ou la prévention du vieillissement cutané.

11. Composition susceptible d'être utilisée pour favoriser la desquamation, conformément aux revendications 1 à 10, **caractérisée en ce qu'**elle comprend une association de β-galactosidase, N-acétyl-glucosaminidase et N-acétyl-galactosaminidase, ladite composition ne comprenant pas de protéase.

12. Composition selon la revendication 11, **caractérisée en ce qu'**elle comprend en plus un produit capable de stimuler l'activité glycosidase.

13. Composition selon la revendication 11 ou 12, **caractérisée en que** les glycosidases favorisant la desquamation représentent entre 0,001 à 15% du poids total de la composition, de manière préférée de 0,01 à 10% et mieux de 0,05% à 5%

14. Composition selon la revendication 12, **caractérisée en qu'**un produit capable de stimuler l'activité glycosidase représente de 0,01 à 10% du poids total de la composition, de manière préférée de 0,1 à 1%.

15. Composition selon l'une quelconque des revendications 12 à 14, **caractérisée en ce qu'**au moins une glycosidase favorisant la desquamation est une glycosidase recombinante.

16. Composition selon l'une des revendications 11 à 15, **caractérisée en ce qu'**elle comprend en plus au moins un autre actif ayant des propriétés favorisant la desquamation.

17. Composition selon l'une des revendications 11 à 16, **caractérisée en ce qu'**elle est appropriée pour l'administration topique.

18. Procédé de traitement cosmétique de la peau pour favoriser la desquamation de la peau consistant à appliquer sur la peau, le cuir chevelu, les ongles et/ou les muqueuses, une composition telle que définie dans les revendications 11 à 17.

19. Procédé cosmétique de traitement des peaux sèches, de la prévention et/ou le traitement du vieillissement cutané consistant à appliquer sur la peau une composition telle que définie dans les revendications 11 à 17.

## Claims

1. Use of a composition for encouraging desquamation, said composition comprising at least a combination of β-galactosidase, N-acetyl-glucosaminidase and N-acetyl-galactosaminidase encouraging desquamation as the active principle, said composition containing no proteases.

2. Use according to claim 1, **characterized in that** the composition further comprises a glycosidase selected from the following enzymes: N-glycanase, cellulases and/or β-glucosidase.

3. Use according claim 1 or 2, **characterized in that** the composition further comprises a product that can stimulate glycosidase activity.

4. Use according to any one of claims 1 to 3, **characterized in that** the glycosidases encouraging desquamation represent in the range 0.001% to 15% of the total composition weight, preferably in the range 0.01 % to 10% and more preferably in the range 0.05% to 5%.

5. Use according to claim 4, **characterized in that** a product that can stimulate glycosidase activity represents 0.01% to 10% of the total composition weight, preferably 0.1 % to 1%.

6. Use according to any one of claims 1 to 5, **characterized in that** at least one glycosidase encouraging desquamation is a recombinant glycosidase.

7. Use according to one of claims 1 to 6, **characterized in that** at least one glycosidase is a glycosidase isolated from the *stratum corneum.*

8. Use according to one of claims 1 to 7, **characterized in that** the composition further comprises at least one other active ingredient having properties that encourage desquamation.

9. Use according to one of claims 1 to 8, **characterized in that** the composition is suitable for topical administration.

10. Use of a composition according to any one of claims 1 to 9, for treating dry skin or for treating and/or preventing cutaneous aging.

11. A composition suitable for encouraging desquamation according to claims 1 to 10, **characterized in that** it comprises a combination of β-galactosidase, N-acetyl-glucosaminidase and N-acetyl-galactosaminidase, said composition containing no protease.

12. A composition according to claim 11, **characterized in that** it further comprises a product that can stimulate glycosidase activity.

13. A composition according to claim 11 or 12, **characterized in that** the glycosidases encouraging desquamation represent in the range 0.001% to 15% of the total composition weight, preferably in the range 0.01 % to 10% and more preferably in the range 0.05% to 5%.

14. A composition according to claim 12, **characterized in that** the product that can stimulate glycosidase activity represents 0.01% to 10% of the total composition weight, preferably 0.1 % to 1%.

15. a composition according to any one of claims 12 to 14, **characterized in that** at least one glycosidase encouraging desquamation is a recombinant glycosidase.

16. A composition according to one of claims 11 to 15, **characterized in that** it further comprises at least one other active ingredient having properties that encourage desquamation.

17. A composition according to one of claims 1 to 16, **characterized in that** it is suitable for topical administration.

18. A method for the cosmetic treatment of the skin to encourage desquamation of the skin, consisting of applying a composition according to any of claims 11 to 17 to the skin, scalp, nails and/or mucous membranes.

19. A cosmetic method for treating dry skin, for preventing and/or treating cutaneous aging, consisting of applying a composition according to any of claims 11 to 17 to the skin.

## Patentansprüche

1. Verwendung einer kosmetischen Zusammensetzung zur Förderung des Abschuppens, die als Wirkprinzip mindestens einen Verbund von β-Galaktosidase, N-Acetylglukosaminidase und N-Acetylgalaktosaminidase, der das Abschuppen fördert, umfasst, wobei diese Zusammensetzung keine Protease umfasst.

2. Verwendung gemäß Anspruch 1, **dadurch gekennzeichnet, dass** die Zusammensetzung außerdem eine Glykosidase enthält, die ausgewählt ist aus den folgenden Enzymen: N-Glykanase, Cellulasen und/oder β-Glukosidase.

3. Verwendung gemäß Anspruch 1 oder 2, **dadurch gekennzeichnet, dass** die Zusammensetzung darüber hinaus ein Produkt umfasst, das in der Lage ist, die Glykosidase-Aktivität anzuregen.

4. Verwendung gemäß einem der Anspruch 1 bis 3, **dadurch gekennzeichnet, dass** die Glykosidasen, die das Abschuppen fördern, zwischen 0,001 und 15% des Gesamtgewichts der Zusammensetzung, vorzugsweise von 0,01 bis 10% und besser von 0,05% bis 5% ausmachen.

5. Verwendung gemäß Anspruch 4, **dadurch gekennzeichnet, dass** ein Produkt, das in der Lage ist, die Glykosidase-Aktivität anzuregen, von 0,01 bis 10% des Gesamtgewichts der Zusammensetzung, vorzugsweise von 0,1 bis 1% ausmacht.

6. Verwendung gemäß einem der Ansprüche 1 bis 5, **dadurch gekennzeichnet, dass** mindestens eine Glykosidase, die das Abschuppen fördert, eine rekombinante Glykosidase ist.

7. Verwendung gemäß einem der Ansprüche 1 bis 6, **dadurch gekennzeichnet, dass** mindestens eine Glykosidase eine Glykosidase ist, die aus dem *Stratum corneum* isoliert ist.

8. Verwendung gemäß einem der Ansprüche 1 bis 7, **dadurch gekennzeichnet, dass** die Zusammensetzung darüber hinaus mindestens einen anderen Wirkstoff umfasst, der das Abschuppen fördernde Eigenschaften besitzt.

9. Verwendung gemäß einem der Ansprüche 1 bis 8, **dadurch gekennzeichnet, dass** die Zusammensetzung zur topischen Anwendung geeignet ist.

10. Verwendung einer Zusammensetzung gemäß einem der Ansprüche 1 bis 9 zur Behandlung von trockener Haut oder zur Behandlung und/oder zur Vorbeugung der Hautalterung.

11. Zusammensetzung, die geeignet ist, gemäß den Ansprüchen 1 bis 10 zur Förderung des Abschuppens verwendet zu werden, **dadurch gekennzeichnet, dass** sie einen Verbund von β-Galaktosidase, N-Acetylglukosaminidase und N-Acetylgalaktosaminidase umfasst, wobei diese Zusammensetzung keine Protease umfasst.

12. Zusammensetzung gemäß Anspruch 11, **dadurch gekennzeichnet, dass** sie darüber hinaus ein Produkt umfasst, das in der Lage ist, die Glykosidase-Aktivität anzuregen.

13. Zusammensetzung gemäß Anspruch 11 oder 12, **dadurch gekennzeichnet, dass** die Glykosidasen, die das Abschuppen fördern, zwischen 0,001 und 15% des Gesamtgewichts der Zusammensetzung, vorzugsweise von 0,01 bis 10% und besser von 0,05% bis 5% ausmachen.

14. Zusammensetzung gemäß Anspruch 12, **dadurch gekennzeichnet, dass** ein Produkt, das in der Lage ist, die Glykosidase-Aktivität anzuregen, von 0,01 bis 10% des Gesamtgewichts der Zusammensetzung, vorzugsweise von 0,1 bis 1% ausmacht.

15. Zusammensetzung gemäß einem der Ansprüche 12 bis 14, **dadurch gekennzeichnet, dass** mindestens ein Glykosidase, die das Abschuppen fördert, eine rekombinante Glykosidase ist.

16. Zusammensetzung gemäß einem der Ansprüche 11 bis 15, **dadurch gekennzeichnet, dass** sie darüber hinaus mindestens einen anderen Wirkstoff umfasst, der das Abschuppen fördernde Eigenschaften besitzt.

17. Zusammensetzung gemäß einem der Ansprüche 11 bis 16, **dadurch gekennzeichnet, dass** sie zur topischen Anwendung geeignet ist.

18. Verfahren der kosmetischen Behandlung der Haut zur Förderung des Abschuppens der Haut, bestehend aus einem Auftragen einer Zusammensetzung, wie sie in den Ansprüchen 11 bis 17 definiert ist, auf die Haut, die Kopfhaut, die Nägel und/oder die Schleimhäute.

19. Kosmetisches Verfahren der Behandlung von trockener Haut, der Vorbeugung und/oder der Behandlung der Hautalterung, bestehend aus einem Auftragen einer Zusammensetzung, wie sie in den Ansprüchen 11 bis 17 definiert ist, auf die Haut.
